# EUROPEAN PATENT APPLICATION

(11) **EP 3 872 168 A1**
(43) Date of publication of application: **01.09.2021**
(21) Application number: 20160158.0
(22) Date of filing: 28.02.2020
(51) Int. Cl.: C12N 5/00, C12N 5/077, C12N 5/074

(54) **CHONDROCYTE DIFFERENTIATION**

(71) Applicant: Cline Scientific AB, 413 53 Mölndal (SE)
(72) Inventor: ANDREASSON, Linnea, 413 04 GÖTEBORG (SE); HOLMQUIST, Niklas, 434 91 Kungsbacka (SE); SUNDH, Patrik, 423 41 Torslanda (SE); EVENBRATT, Hanne, 443 95 Stenkullen (SE)
(74) Representative: Ström & Gulliksson AB

(57) **Abstract**

Disclosed is a method for differentiating induced pluripotent stem cells (iPSCs) into chondrocytes. The method comprises the steps of seeding a surface of a substrate with iPSCs. The surface is coated with nanoparticles in a particle density of at least 500 particles/µm², and parts of the surface in between said nanoparticles are coated with a coating agent. Growth differentiation factor 5 (GDF5) molecules are attached to the nanoparticles. The method further comprises the steps of adding a first differentiation medium to the seeded iPSCs, and allowing the seeded iPSCs to differentiate at least into chondrocyte progenitor cells on the surface in the presence of the first differentiation medium.

## Description

### Technical Field of the Invention

The invention relates to a method for differentiating stem cells into chondrocytes, a substrate for differentiating said stem cells into chondrocytes and a bone or cartilage implant comprising chondrocytes differentiated by said method. Further, the invention relates chondrocytes obtainable by differentiating stem cells into chondrocytes.

### Background

There are several conditions, such as trauma or the disease Osteoarthritis (OA), which degenerates cartilage and/or bone structure of the patient. OA specifically is a type of joint disease that results from breakdown of joint cartilage and underlying bone. In many cases, no cures exist for such cartilage and bone impairing diseases, which may lead to the need for surgical procedures to exchange the damaged tissue for a bone or cartilage implant. The human body cannot reproduce or repair cartilage on its own, since cartilage, unlike other tissues, lacks its own blood supply. Therefore, damaged cartilage has to be replaced either by implants or regenerated by the implantation of healthy cartilage cells.

Autologous chondrocyte implantation (ACI) technology has emerged over the past decade as a disease-modifying treatment with satisfying clinical results among patients suffering from isolated cartilage injuries. However, the method is complicated and very time consuming. It has therefore been considered to use stem cells of various origin to be differentiated into chondrocytes in order to not be restricted to an autologous source and to be able to expand a limited number of cells into chondrocytes for implantation.

Cartilage is evolved through chondrogenesis, and arises from chondrocyte differentiation from mesenchymal stem cells and chondroprogenitor cells in early development. The differentiation process is initiated by mesenchymal condensation, meaning reduction of intercellular spaces and is followed by continued differentiation. Molecules such as growth differentiation factor 5 (GDF5), transforming growth factor beta 1 (TGFβ-1), and transforming growth factor beta 3 (TGFβ-3) are known to be essential during chondrogenesis. Decreased levels of these morphogens are suggested to be involved in diseases such as i.e. osteoarthritis (OA).

Humans are multicellular creatures, which like all multicellular organisms are formed from one single cell, the fertilized egg. A challenge during development is how to generate distinct cell types and organs from a single cell. Organs and tissue such as cartilage and limbs are formed during development due to orchestration of growth factors that functions as morphogens, such as GDF5 and TGFβ-1 or TGFβ-3.

In addition to the field of implants, various methods for controlling differentiation of stem cells have been explored, and the use of morphogenic proteins have been used in experiments in attempts to regulate i.e. the formation of tenocytes, chondrocytes and osteoblasts. Such research target the possibility of helping patients suffering from diseases or conditions impairing their cartilage or bone formation. For instance, by deriving induced pluripotent stem cells (iPSCs) from chondrocytes, it may be possible to use an allogenic stem cell line as a donor to patients in need of treatment.

In WO 2010/051032 methods and devices for stimulating mesenchymal stem cells in a stem cell source to differentiate into osteoblasts capable of forming bone are disclosed. In WO 2013176538, a culture medium for promoting human bone marrow mesenchymal stem cells differentiation into tenogenic lineage comprising GDF5 is disclosed, and in WO 2016/193175, a method for producing a cellular composition with *in vivo* bone and/or cartilage forming potential is disclosed.

However, the above mentioned prior art suffer from several disadvantages. A method displaying increased regulation and a higher reproducibility, would be preferable.

Hence, there is need for an improved method for achieving differentiation of stem cells into chondrocytes, in order to be able to induce the formation of new cartilage in an efficient and reproducible manner, and to be able to provide cartilage and bone implants.

### Summary

Consequently, the present invention seeks to mitigate, alleviate, eliminate or circumvent one or more of the above-identified potential deficiencies in the art and disadvantages singly or in any combination by providing a method for differentiating induced pluripotent stem cells (iPSCs) into chondrocytes.

Such a method comprises the steps of seeding a surface of a substrate with iPSCs. The surface is coated with nanoparticles in a particle density of at least 500 particles/µm², and parts of the surface in between the nanoparticles are coated with a coating agent, and growth differentiation factor 5 (GDF5) molecules are attached to the nanoparticles. The method further comprises the steps of adding a first differentiation medium to the seeded iPSCs and allowing the seeded iPSCs to differentiate at least into chondrocyte progenitor cells on the surface in the presence of the first differentiation medium.

In one embodiment, the particle density is less than 1500 particles/µm².

In one embodiment, the method further comprises removing formed condensed chondrocyte progenitor cell aggregates from the substrate surface with GDF5 attached thereto and further differentiating the removed condensed chondrocyte progenitor cell aggregates into chondrocytes, in a second differentiation medium. This allows the condensed chondrocyte progenitor cell aggregates to differentiate into chondrocytes.

In one embodiment, the first and second differentiation medium both comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof.

In a second aspect of the invention, there is provided a chondrocyte obtainable by the method for differentiating iPSCs into chondrocytes disclosed herein above.

In a third aspect of the invention, there is provided a solid substrate for differentiating induced pluripotent stem cells (iPSCs) into chondrocytes. The substrate comprises a substrate surface coated with nanoparticles linked to the surface a particle density of at least 500 particles/µm², a coating agent coating the parts of the surface between the nanoparticles on substrate surface and growth differentiation factor 5 (GDF5) molecules attached to the nanoparticles.

Other objectives, features and advantages of the present invention will appear from the following detailed disclosure, from the attached claims, as well as from the drawings. It is noted that the invention relates to all possible combinations of features.

Generally, all terms used in the claims are to be interpreted according to their ordinary meaning in the technical field, unless explicitly defined otherwise herein

### Detailed Description of Preferred Embodiments of the Invention

As mentioned herein above, the human body cannot reproduce cartilage tissue when such tissue has been damaged. Hence, for most conditions causing breakdown of cartilage there is no cure available. Damaged cartilage may be replaced by implants added surgically, or cartilage can be regenerated by the implantation of healthy cartilage cells. However, the methods presently available are time consuming and cumbersome. Furthermore, current methods for providing implantable chondrocytes (cartilage cells) often lack reproducibility and are hard to regulate, generating chondrocytes of varying quality and properties.

The present inventors envisaged that healthy chondrocytes could be produced, with high reproducibility and in a controlled way, from induced pluripotent stem cells (iPSCs) differentiating into chondrocytes in the presence of differentiation factors, *inter alia* the protein growth differentiation factor 5 (GDF5), on a substrate surface and later in a pellet formation. In short, various differentiation factors were attached to nanoparticles coated on the substrate surface in a gradient pattern. This allowed for analysis of the behaviour of iPSCs seeded to said surface at different particle densities, and thereby at different concentrations of the differentiation factor.

As previously known, distinct cell types and organs in the human body are derived from a single cell. One theory on how this is achieved is that biomolecules provide signals, which induce different cellular responses. Molecules in the transforming growth factor beta (TGFβ) family are a type of signalling biomolecule, and GDF5, TGFβ-1, and TGFβ-3 are known to affect chondrogenesis (Goldring M. B. 2012).

During chondrogenesis, cartilage is generated from chondrocyte differentiation from mesenchymal stem cells and chondroprogenitor cells in early development. Chondroprogenitor cells are cells which have partly differentiated into chondrocytes. The differentiation process is initiated by mesenchymal condensation, meaning reduction of intercellular spaces and is followed by continued differentiation.

Even though GDF5 and TGFβ molecules have previously been used in methods for differentiating stem cells into chondrocytes, there is however still a need for further methods providing increased regulation and more local and exact stimulation of the stem cells, in order to provide for differentiation with high reproducibility, in a controlled way. An issue with inducing differentiation of stem cells in various liquid medias is that controlled stimulation is hard to achieve. The stem cells in such liquid media will receive a more random stimulation from different differentiation inducing molecules depending on where they are present in the volume of the media, than if the stem cells are bound to a surface. As a result, differentiation using biomolecules such as GDF5 and TGFβ molecules to stimulate stem cells to develop into chondrocytes in a liquid medium is unpredictable and the result is hard to foresee.

The present inventors have thus elucidated a method for determining the impact of different concentrations of different biomolecules (GDF5, TGFβ-1 and TGFβ-3) on chondrocyte derived iPSCs (c-iPSCs) by using a gradient surface of gold nanoparticles to which the biomolecules were bound. It was surprisingly found that the protein growth differentiation factor 5 (GDF5) attached to the nanoparticles at a given particle density of the gradient pattern, resulted in a preferred differentiation behaviour of the seeded iPSCs. Corresponding effects were not seen with other differentiation factors (e.g. TGFβ-1 or TGFβ-3). The effect could subsequently be reproduced with nanoparticles at a particle density within the identified range homogenously attached to the substrate surface. The local concentration of GDF5 on the substrate surface is significantly higher than the concentration that may be used in solution. Without being bound to any theory this may, at least partly, explain the result seen.

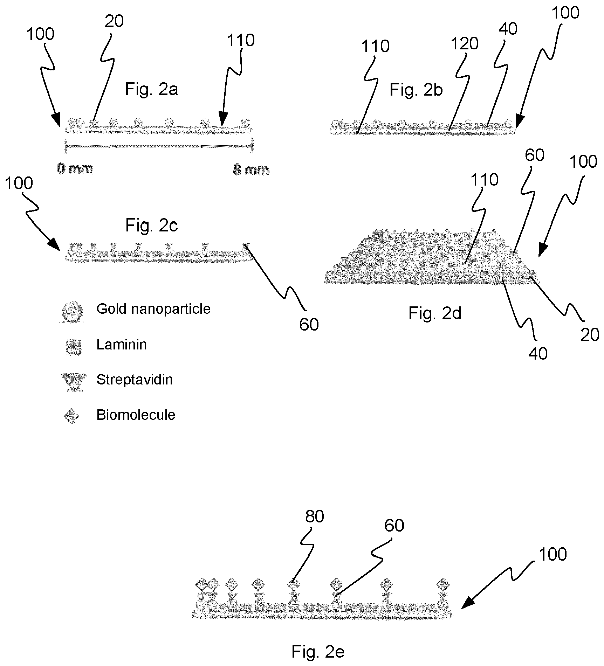

In short, to create gradient surfaces a glass substrate 100 was coated with gold nanoparticles 20 in a gradient pattern (Fig. 2a) and laminin 40 was used to cover parts 120 between the nanoparticles 20 (Fig. 2b). Streptavidin 60 was attached to the gold particles 20 (Figs 2c and 2d), and biomolecules 80 (*inter alia* GDF5) were biotinylated and attached to the streptavidin 60, whereby a surface having a gradient of biomolecules were obtained (Fig. 2e). Subsequently, c-iPSCs were seeded to the surfaces (not shown). The purpose of using gold nanoparticles was to enable binding of signalling biomolecules in a controlled and continuous gradient pattern to a stable substrate (see *"Preparation of gradient surfaces of GDF5, TGFβ-1 and TGFβ*-*3*" of the experimental part for more details regarding the method).

More specifically, the inventors surprisingly found that the area where nanoparticles were present at a particle density of at least 500 particles/µm² and GDF5 attached thereto gave a better pre-differentiation result compared to other particle densities of nanoparticles (resulting in another concentration of GDF5). In the particle density range of 500 to 1500 particles/µm² on the substrate with GDF5 attached thereto, the seeded c-iPSCs formed so called budding zones, in which condensed cell clusters were formed. Outside this range, budding zones were not present. Since the differentiation process of iPSCs into chondrocytes is initiated by mesenchymal condensation, meaning reduction of intercellular spaces and is followed by continued differentiation, the formation of budding zones with cell clusters/aggregates is an indication of initiation of chondrogenesis and evolvement into chondrocyte progenitor cells and eventually chondrocytes.

After pre-differentiation on the substrate surface, progenitor chondrocyte cells were obtained, which could be further differentiated as a pellet suspended in a second differentiation medium, whereby chondrocytes were obtained. Various controls were used to verify the effect and the results, as will be explained more in the following.

Homogenous density surfaces (h.d. surfaces) having gold nanoparticles within the range 500 to 1500 particles/µm² and GDF5 attached thereto were subsequently reproduced for further analysis (disclosed in the section *"Differentiation on gradient surfaces and h.d. surfaces, and c-iPSC analysis on gradient surfaces and h.d. surfaces* " of the Experimental part). The gradient surfaces coated with TGFβ-1 or TGFβ-3 did not give any satisfying results at any particle density of the gold nanoparticles at the gradient surface.

Hence, an embodiment of the invention relates to a method for differentiating induced pluripotent stem cells (iPSCs) into chondrocyte cells. The differentiation takes place in a stable and controllable environment, and provides a continuously foreseeable result.

The method will be explained more detailed in the following, with reference to the Figures 2a-2e and comprises seeding a surface 110 of a substrate 100 with iPSCs, when the surface 110 is coated with nanoparticles 20 having a particle density of at least 500 particles/µm². The parts 120 of the surface 110 in between the nanoparticles 20 are coated with a coating agent 40, and GDF5 molecules are attached to the nanoparticles 20. A first differentiation medium is added to the seeded iPSCs and the seeded iPSCs are then allowed to differentiate at least into chondrocyte progenitor cells on the surface 110 in the presence of the first differentiation medium.

Hence, the method may comprise using either nanoparticles coating the glass substrate surface 110 in a gradient pattern, or having nanoparticles coating the surface 110 homogenously. The nanoparticle gradient surface results in a gradient pattern of GDF5 molecules attached thereto, and a homogenous density of nanoparticles results in that GDF5 has a homogenous density when attached thereto. According to an embodiment, the nanoparticles coat the glass substrate surface 110 homogenously.

Chondrocyte progenitor cells are cells, which have differentiated from iPSCs into a pre-stage of chondrocyte cells. They have a constrained differentiation potential and a lower capacity for self-renewal indicating a more mature stage than iPSCs and are a key stage in the differentiation into chondrocytes.

The coating agent 40 may be a proliferative agent, such as laminin used in the experiments disclosed herein. However, other proliferative agents, such as an extra cellular matrix proteins (ECM proteins), e.g. laminins and fibronectins, and cell specific cytokines, or a combination thereof may be used for the method disclosed herein. The laminin used herein does not only cover and thereby block the parts 120 between the gold nanoparticles 20, such that unwanted attachment of the biomolecules to the surface 110 is prevented; the laminin also, at least to some extent, stimulate cell differentiation.

Further, it has been shown that the c-iPSCs have an impaired capacity to attach to glass surfaces. Laminin therefore also served as an agent which successfully improved cell adhesion to the substrate.

Hence, coating agents in general have several purposes, e.g. enhancing binding properties of cells to glass surfaces and preventing unwanted adhesion of biomolecules to substrate surface instead of binding to wanted particles (in this case, the streptavidin coated gold nanoparticles).

The iPSCs may be differentiated on the substrate surface 110, for between 3 and 10 days, preferably between 4 and 8 days. The upper time limit of 10 days for the differentiation on the substrate surface is limited by the adherence of the iPSCs to the substrate 100. After 10 days, it is hard to maintain the iPSCs on the substrate surface 110. As such, the differentiation on the substrate surface may continue as long as the cells are able to be maintained at the substrate. The lower limit of 3 days is the preferred minimum time required for the differentiation on the substrate surface 110. Less than 3 days will probably not result in any differentiation occurring.

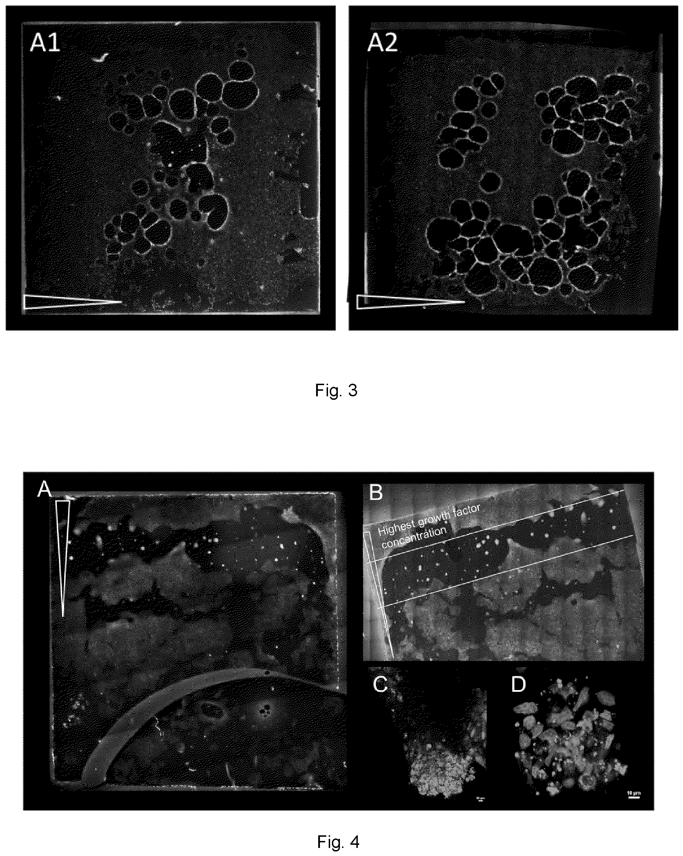

After a few days, such as 5 days as applied in the experimental example disclosed in the result section of the part *"Differentiation on gradient surfaces and h.d. surfaces, and c-iPSC analysis on gradient surfaces and h.d. surfaces*", the cells will form cell aggregates, also referred to as clusters herein, in budding zones on the substrates. The term budding zone describes the appearance of the surfaces and a zone in which cell clusters form cell buds or nodular structures, which are separated from a larger crowd of cells. For instance, in Fig. 4 in section B, a light grey area form a carpet like structure of cells. The white dot like formations are condensed cell clusters (nodular structures/nodules) and at the end of the light grey area within the white lines, a budding zone is shown. At a top of a triangular shaped light grey area within the white lines, a white separate nodule/bud is about to bud off within the budding zone. The budding zones are of interest since the formation of the cell aggregates in the budding zones indicate an initiation of chondrogenic behaviour.

An embodiment thus relates to said method which further comprises removing formed condensed chondrocyte progenitor cell aggregates from the substrate surface 110 having GDF5 attached thereto. Once removed, the condensed chondrocyte progenitor cell aggregates are further differentiated in a second differentiation medium. According to such an embodiment, the further differentiation is preferably performed for 4 to 10 weeks, such as for 5 to 8 weeks. Further differentiation for less than 4 weeks have low likelihood of resulting in obtained chondrocytes.

Preferably, the removed condensed cell aggregates are formed into a three dimensional pellet structure, before being further differentiated. The removed condensed cell aggregates were combined into a pellet structure since further condensation increases a cartilage like structure and induces secretion of cartilage-specific matrix. The removal of the condensed chondrocyte progenitor cell aggregates may be conducted by releasing the cells from the gradient surface and h.d. surface and a separation of the condensed chondrocyte progenitor cell aggregates, followed by centrifuging the condensed chondrocyte progenitor cell aggregates, such that the pellet structure is obtained. The cells may be released by adding trypsin. Once released, the action of trypsin may be quenched by adding human serum. Details regarding obtaining such pellets and further differentiation is disclosed in *"Pellet cultures from GDF5 gradient surfaces, GDF5 h.d. surface and chondrocyte control experiments".*

The first and second differentiation medium may be of the same kind. An exemplary composition of the differentiation medium is disclosed in Table 1 herein below. The first and/or the second differentiation medium, may according to an embodiment comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof. Other differentiation medium known in the art may also be used. The differentiation medium provides additional factors, *inter alia* TGFβ-1 and TGFβ-3, of importance for cell growth and differentiation. The differentiation medium may comprise 2 to 30 ng/mL, such as 5 to 15 ng/mL, preferably 10 ng/mL of TGFβ-1 and/or 2 to 30 ng/mL, such as 5 to 15 ng/mL, preferably 10 ng/mL of TGFβ-3.

According to an embodiment, a maintenance medium is added to the substrate surface 110 after the iPSCs have been seeded to the surface 110. The maintenance medium is preferably added before the differentiation medium is added. The maintenance medium may for instance be *Cellartis® DEF-CS*™ *500 Basal Medium with Additives.* However, any other known maintenance medium known in the art may be used. The purpose of the maintenance medium is to maintain the viability and physiological characteristics of the cell culture until they have attached to the surface and are ready to start the differentiation process.

The iPSCs used herein may be c-iPSCs (chondrocytes derived iPSCs) generated by reprogramming chondrocytes using a preferably footprint-free method based on mRNA delivery. Stem cells are cells that can differentiate into other types of cells, and can also divide in self-renewal to produce more of the same type of stem cells. Mammals comprise two types of stem cells being embryotic stem cells and adult stem cells, the latter also referred to as somatic stem cells. Somatic stem cells are derived from tissue samples from (healthy) adults. iPSCs are pluripotent stem cells, meaning they may differentiate into different types of cells, thus being a promising starting point for regenerative medicine. Since iPSCs may be derived from somatic stem cells, there is no need for the use of embryotic stem cells, though embryotic stem cells may be used. According to an embodiment, the iPSCs are not derived from embryotic stem cells. As known in the art, iPSCs may be formed by exposing cells, such as chondrocytes, to reprogramming factors, which reprograms the cells into iPSCs.

The culturing of cells disclosed herein is explained in the section *"Cell culturing"* of the Experimental part. An advantage in using chondrocytes and reprogramming them to c-iPSCs is that iPSCs have been shown to more easily differentiate along lineages related to the cell type of origin, probably due to a residual epigenetic memory (Borestrom, C., S. Simonsson, et al 2014). In addition, the ethical issues concerning human embryonic stem cells (hESCs) are avoided since chondrocytes for reprogramming can be obtained by healthy individuals.

In one embodiment, 20 000 to 100 000 iPS cells/cm², preferably 30 000 to 70 000 iPS cells/cm², most preferred 50 000 cells/cm², are seeded to the substrate surface 110.

In the experiments disclosed herein, the nanoparticles 20 are gold particles coated with thiolated streptavidin, and the GDF5 molecules are biotinylated and attached to the nanoparticles through biotin/streptavidin interaction. However, any such known principle for facilitating binding and interaction between nanoparticles and a biomolecule may be used, since the biotin/streptavidin interaction is only an interaction according to one embodiment. For instance, such interaction may be formed using an ion-exchange/interaction, hydrophobic interactions and dative binding, covalent binding of thiols, carbo di-imide chemistry, bi-functional linkers (such as EDC/NHS chemistry) or click chemistry.

Alternatively, the molecules may be purchased already provided with a biotin or another type of interaction marker. The method used for the biotinylation is disclosed in the section of the Experimental part. In addition, other methods for biotinylation known in the art may be used.

In tissue engineering, scaffolds are designed to provide an environment for the formation of regenerated viable tissue. Scaffolds tend to mimic an extracellular matrix of a certain tissue, and are engineered to cause preferably cellular interactions stimulating cell growth. Tissue scaffolds can be formed of different materials, for instance natural or synthetic materials, several used materials are biodegradable. An embodiment thus relates to integrating obtained chondrocytes into a matrix and/or scaffold. Such integration may cause the chondrocytes to further divide and reproduce themselves and form a biological implant which may replace damaged cartilage or bone tissue in a subject. Hence, the present disclosure also concerns a bone or cartilage implant, made of a scaffold and/or matrix comprising chondrocytes which have been obtained by differentiating iPSCs into chondrocytes by the method disclosed herein.

Further, the chondrocytes obtained by differentiating iPSCs into chondrocytes are useful in chondrocyte implantation. Hence, an embodiment relates to a chondrocyte obtainable by differentiating iPSCs into chondrocytes by the method disclosed herein.

Without further elaboration, it is believed that one skilled in the art can, using the preceding description and the following experimental part, utilize the present invention to its fullest extent. The preferred specific embodiments described herein are, therefore, to be construed as merely illustrative and not limitative of the remainder of the description in any way whatsoever. Further, although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc. do not preclude a plurality.

### Brief description of the Drawings

By way of example, embodiments of the present invention will now be described with reference to the accompanying drawings, in which:
Fig. 1a shows a montage of SEM pictures taken at different positions with 1 mm interval along a gold nanoparticle gradient surface, the scale bar indicates 200 µm. The gradient is verified up to 8 mm on a 18x18 mm glass surface;
Fig. 1b shows a diagram of the gradient profile for a gold nanoparticle gradient functionalized with GDF5. The x-axis indicates gradient distance in mm and the y-axis shows GDF5 particles/µm² as a function of the gradient distance;
Fig. 2a shows a schematic glass surface being 8 mm wide and provided with gold nanoparticles attached in a gradient pattern on a glass substrate;
Fig. 2b shows the schematic glass surface of Fig. 2a, having Laminin molecules attached to the glass surface between the gold nanoparticles, to avoid unwanted adherence of other molecules to the glass surface between each gold nanoparticle;
Fig. 2c shows the schematic glass surface of Fig. 2b having Streptavidin attached to the gold nanoparticles. The Streptavidin will act as a linker to biotinylated biomolecules;
Fig. 2d shows a perspective view of the schematic glass surface of Fig. 2c. The gradient of Streptavidin coated gold nanoparticles is ready to be functionalized by biotinylated morphogens;
Fig. 2e shows a functionalised glass substrate surface, where biotinylated biomolecules have been attached to the Streptavidin. In the present disclosure, said biomolecules are GDF5, TGFβ-1 and TGFβ-3;
Fig. 3 shows laminin control gradient surfaces (A1, A2). The gradient surfaces were visualized with IN CELL Analyzer 6000 (IN Cell 6000, GE Healthcare, United Kingdom). Cavities with no cells in the centre were formed, mainly at higher laminin densities. The bars on each surface A1, A2 indicate the direction of the nanoparticle density gradient with laminin coupled to the surface;
Fig. 4 shows the budding zone in (A) a GDF5 gradient surface, 18x18 mm, seeded with c-iPSCs visualized using high throughput confocal IN CELL Analyzer 6000. The bar on the left upper side indicates the extent of the gradient where the continuous density increase is shown with a marker on the left-hand side, with low GDF5 density at the bottom of the image and high GDF5 density at the top, (B) focusing on the budding cell clusters identified at particle density of 500-1500 gold nanoparticles/µm². Two white lines indicate the budding zone, and (C-D) show close up pictures of buds identified in the budding zone shown in (B), the scale bars in (C) and (D) are 10 µm;
Fig. 5A-F all show comparative pictures on cell behavior on GDF5, TGFβ-1 and TGFβ-3 h.d. surfaces, 18x18 mm, visualized using high throughput confocal IN CELL Analyzer 6000;

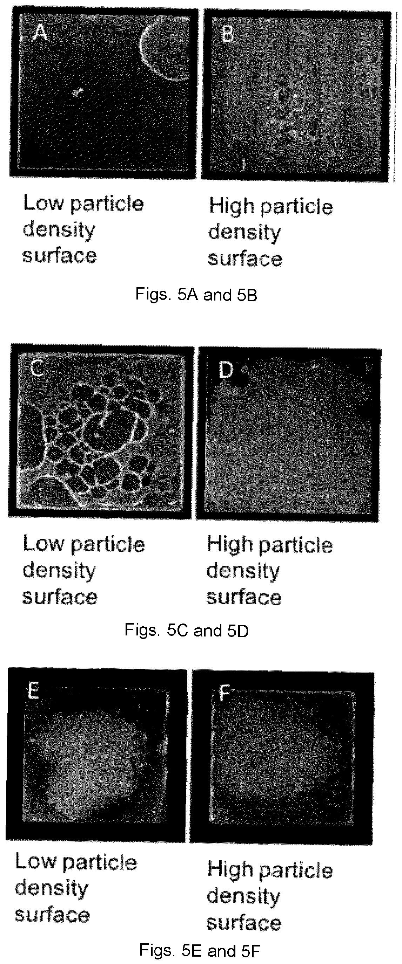

Fig. 5A shows GDF5 surfaces with a low particle density at 400 particles/µm². No budding zones are identified;
Fig. 5B shows a GDF5 surface with a particle density in the range of the budding zone at 900 particles/µm². Buds are clearly identified as brighter colored clusters;
Fig. 5C shows TGFβ-1 surfaces with a low particle density at 600 particles/µm². Cell-free cavities were formed, a response comparable to cells on laminin only (Fig. 3);
Fig. 5D shows evenly distributed cells on TGFβ-1 surfaces with a high particle density of 2000 particles/µm²;
Fig. 5E shows TGFβ-3 surfaces with low density, 600 particles/µm², and Fig. 5F shows TGFβ-3 surfaces with high density, 1900 particles/µm²;

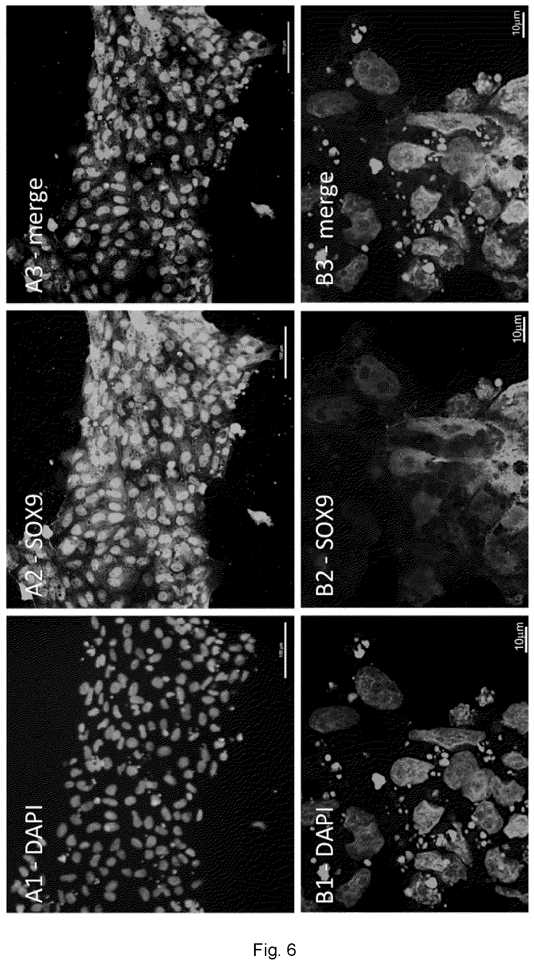

Fig. 6 shows immunostaining for SOX9 in c-iPSCs after five days of differentiation on a GDF5 gradient. (A1-A3) Images were acquired in the budding zone using florescence microscopy, 20X objective. Scale bars denote 100 µm. (B1-B3) Images were acquired in the budding zone with a confocal microscope, 40X objective. Scale bars denote 10 µm;

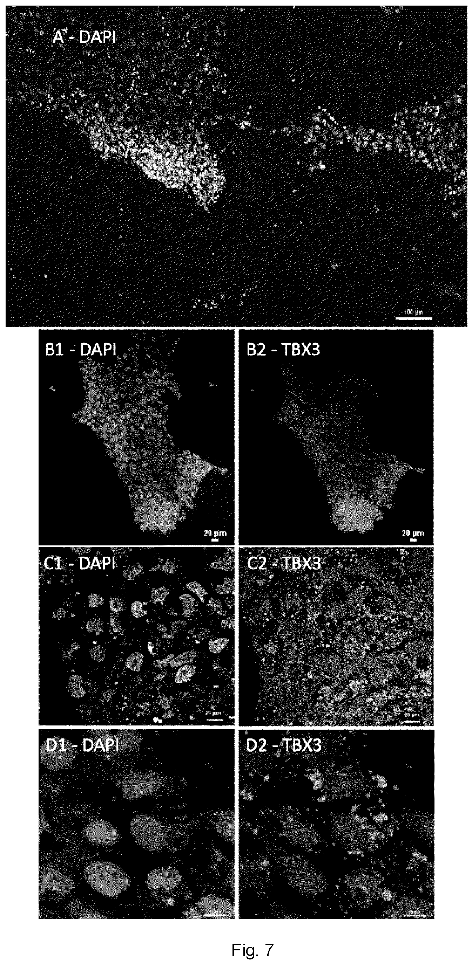

Fig. 7 shows that TBX3 are localized in the nuclei and in vesicles in the budding zone. TBX3 expression is visualized after five days of differentiation on a GDF5 gradient. (A) shows a budding zone on a GDF5 gradient. Scale bar 100 µm. (B1-B3) and (C1-C3) show two different budding clusters. Images were acquired with a confocal microscope, 40X objective. Scale bars 20 µm. (D1-D3) is a closeup of a budding cluster. Images were acquired using a 60X objective. Scale bars 10 µm.

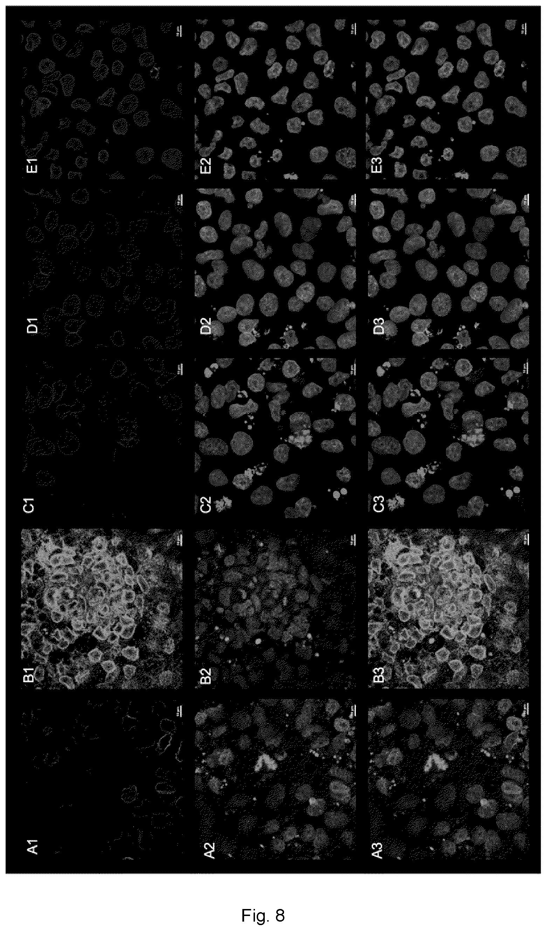

Fig. 8 shows iPSCs on h.d. surfaces immunostained with TBX3 monoclonal (Table 2) antibody, using confocal microscopy. TBX3 expression is clearly upregulated on the GDF5 h.d. surface with a particle density in the range of the budding zone, compared to all other surfaces. (A) shows a GDF5 surface with low particle density. (B) is a GDF5 budding zone surface. (C) shows a TGFβ-3 surface with the same particle density as (B). (D) is a TGFβ-3 surface with the same particle density as the picture (B) but with GDF5 (10 ng/ml) added in the differentiation medium. (E) is a surface without biomolecules, coated with Coat-1 (DEF-CS 500 Coat-1, Cellartis, Sweden); and

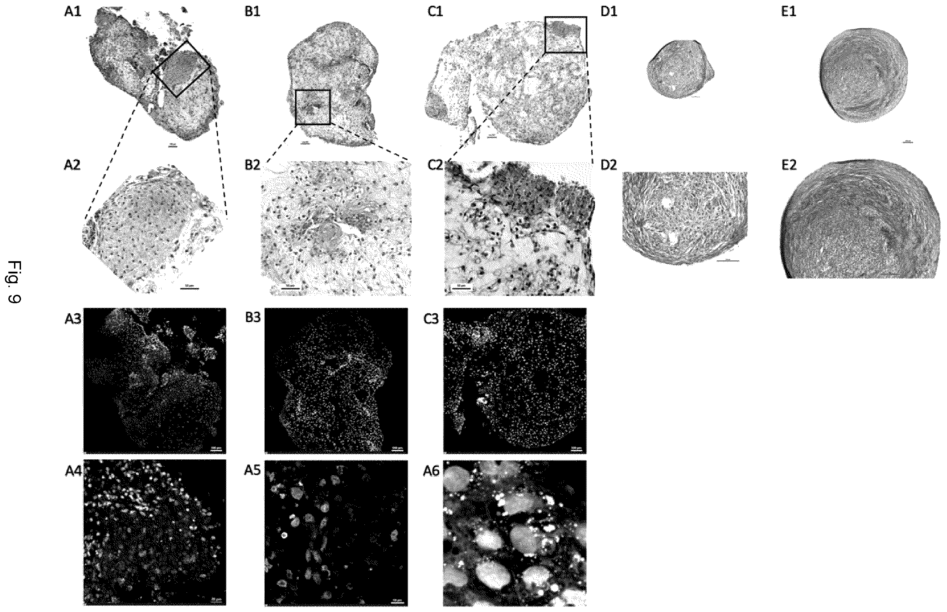

Fig. 9 shows histological sections of pellets generated from c-iPSCs (A-C) and chondrocytes (D-E). A1, A2 and E1, E2 were stained with Alcian Blue van Gieson that demonstrates the presence of proteoglycans and collagen. A3-A6 & B3-C3 were immunostained for TBX3 which were especially upregulated in the areas with higher amounts of GAG. (A1-A6) show c-iPSCs pre-differentiated on a GDF5 h.d. surface with low particle density. (B1-B3) show c-iPSCs pre-differentiated on GDF5 h.d. surface with budding zone particle density. (C1-C3) are c-iPSCs pre-differentiated on GDF5 gradient surface. (D1-D2) show chondrocyte pellet pre-differentiated on GDF5 gradient surface. (E1-E2) show a chondrocyte pellet with 5% human serum in the medium. In A1-E1, A3-C3, A5-A6, the scale bars indicate 100 µm. In A2-E2, the scale bars indicate 50 µm. In A4, the scale bar indicates 20 µm.

### Experimental

The following examples are mere examples and should by no means be interpreted to limit the scope of the invention, as the invention is limited only by the accompanying claims.

### Abbreviations

- OA: Osteoarthritis
- ACI: autologous chondrocyte implantation
- c-iPSc: chondrocyte derived induced pluripotent stem cells
- TGFβ-1: transforming growth factor beta 1
- TGFβ-3: transforming growth factor beta 3
- GDF5: growth differentiation factor 5
- BMP14: bone morphogenetic protein 14
- BMPs: bone morphogenetic proteins
- ECM: extracellular matrix
- TBX3: T-box transcription factor 3
- DAPI: 4',6-diamidino-2-phenylindole
- PBS: phosphate buffered saline
- GAGs: glycosaminoglycans
- SOX9: Transcription factor SOX9
- PDGF: platelet-derived growth factor
- h.d. surface: homogenous density surface

### Cell culturing

### Method - generating c-iPSCs from chondrocytes

It has previously been shown that chondrocytes from autologous chondrocyte implantation (ACI)-donors can be reprogrammed into iPSCs using a footprint-free method based on mRNA delivery (Borestrom, C., S. Simonsson, et al 2014), a method that was applied to generate c-iPSCs for this study. The iPSCs were derived from chondrocytes from an anonymous female donor.

In short, the method involves obtaining chondrocytes form ACI-donors, isolating said chondrocytes and expanding them in a chondrocyte medium. Non-integrating mRNA reprogramming technology was used and mRNA reprogramming was conducted using the Stemgent mRNA Reprogramming Kit according to the manufacturer's instructions, with some minor modifications, performing daily mRNA transfections during 21 days. Clonal iPSC lines were established by picking hESC-like colonies.

### Method - cell culturing of c-iPSCs for control experiments

The iPSCs were stored in liquid nitrogen at -196°C until use. Newly thawed c-iPSCs were seeded for monolayer culturing in cell culture flasks (Corning™ Primaria™ Tissue Culture Flasks, vented, FisherScientific, USA) which were coated specifically for c-iPSCs (DEF-CS 500 Coat-1, Cellartis, Sweden) and placed in a 37°C humidity chamber at 90% humidity and 5% CO2. Cell medium (Cellartis® DEF-CS™ 500 Basal Medium with Additives, Cellartis, Sweden), supplemented with additional growth factors, was changed every day and cell passage was performed every third day. All handling of cells was performed in a sterile cell lab area.

### Method - cell culturing of chondrocytes for control experiments

The chondrocytes used for the pellet-experiments were provided from three combined anonymous male donors, with written consent. Chondrocytes were expanded in a monolayer in chondrocyte medium consisting of Dulbecco's modified Eagle's medium/F12 (DMEM/F12, ThermoFisher Scientific, USA) supplemented with 0.5 mL 8 mM L-ascorbic acid (Sigma Aldrich, USA), 1% penicillin-streptomycin (Sigma Aldrich, USA) and 10% human serum, at 37°C in 5% CO2 and 90% relative humidity. Medium was changed three times per week. All handling of cells was performed in a sterile cell lab area.

### Preparation of gradient surfaces of GDF5, TGFβ-1 and TGFβ-3

### Method

Cline Scientific's Nano Gradients and Nano Surfaces (Cline Scientific AB, Sweden) were used as a tool in the differentiation process. The gradient surface may be a nano gradient surface as disclosed in EP 2 608 896 B1. Such nano gradient surface has a continuous gradient of deposited and electrically charged nanoparticles. The nanoparticles preferably have an average diameter between 10 and 60 nm, and the average centre-to-centre distance of the nanoparticles is typically about 10 to 60 nm in one end of the gradient and about 100 to 150 nm in the other end of the gradient.

A particle density of nanoparticles on the gradients were in the range of from 3 to 3000 particles/µm², determined with Scanning Electron Microscopy (SEM) using a Zeiss Ultra 55 operating at an accelerating voltage of 3.00 kV. Images were acquired in the secondary/backscattered electron mode using the In Lens detector. The nanoparticles used were gold nanoparticles and the surface was a glass surface. The substrate 100 is shown schematically in Figs. 2a-2e.

According to a functionalization protocol, thiolated streptavidin 60 (SH-streptavidin, ProteinMods, USA) was applied to the gold nanoparticles 20, as shown in Fig. 2c. After sufficient incubation in room temperature, superfluous streptavidin 60 was rinsed off with Phosphate Buffered Saline (PBS) (Phosphate Buffered Saline, Amresco, USA).

Thereafter, laminin 521 (Biolaminin 521 LN, BioLamina, Sweden) was applied to coat parts 120 of the glass surfaces 110 between the nanoparticles. After incubation (as above), superfluous laminin 40 was rinsed off with PBS. The substrate 100 and its prepared surface 110 having a gradient of gold nanoparticles 20, having thiolated streptavidin 60 attached to said nanoparticles 20, and where the surfaces 110 in between the nanoparticles 20 are coated with laminin 40 is shown in Fig. 2c and 2d).

Previous experiments with glass surfaces showed that the c-iPSCs have an impaired capacity to attach to glass. Therefore, laminin was attached to the gradient surface between the biomolecule functionalized particles, successfully ameliorating cell adhesion (Aumailley, M. 2013). In addition, laminin will overall enhance the binding of iPSCs to the surface and is thought to stimulate cell differentiation.

GDF5 (R&D Systems, Bio-Techne, MN, USA), TGFβ-1 (R&D Systems, Bio-Techne, MN, USA) and TGFβ-3 (R&D Systems, Bio-Techne, MN, USA) were biotinylated to facilitate binding to streptavidin. The biotinylation was performed with a superfluous amount of biotin (EZ-LinkTM Sulfo-NHS-LC-Biotin, Thermo Fischer Scientific, USA) to make sure that biotin was attached to all available sites of the proteins GDF5, TGFβ-1 and TGFβ-3.

The excess amount of biotin that was not attached was discarded using repeated wash and centrifugation according to Thermo Fisher's biotinylation protocol. The success of the biotinylation was controlled using a HABA test (HABA/Avidin Reagent, Sigma Aldrich, USA). The HABA test ensured that the number of biotins attached to the proteins were within specified limits according to the producers.

The biotinylated proteins 80 with a known concentration, 40nM in suspension, were attached to the streptavidin 60 coated gold nanoparticles 20 and incubated at 4°C overnight. Similarly to the previous steps, superfluous proteins (GDF5, TGFβ-1 and TGFβ-3) were rinsed off with PBS. After functionalisation the surfaces were stored in 4°C until c-iPSCs were seeded and differentiated on the gradient surfaces. A functionalised gradient surface 100 is shown schematically in Fig. 2e.

During SEM analysis of the gradient surfaces, the particle density where the iPS cells had a response that was of interest were identified (500 to 1500 particles/µm²). Densities within the range of interest were reproduced on new homogenous density (h.d.) surfaces (without a gradient pattern) (Nano Surfaces, Cline Scientific AB, Sweden).

H.d. surfaces with a particle density lower (400 particles/µm²) than the particle density of interest (500 to 1500 particles/µm²) were used as controls.

Gradient surfaces with only streptavidin and laminin were used as reference surfaces.

Several h.d. surfaces within the range of 500 to 1500 particles/µm² were reproduced and tested, among others 575 particles/µm², 652 particles/µm², 729 particles/µm², 768 particles/µm², 792 particles/µm², 859 particles/µm², 1017 particles/µm², 1027 particles/µm², 1083 particles/µm², 1145 particles/µm², and 1367 particles/µm².

The h.d. surfaces and control surfaces were prepared using the same method as described above.

### Results

Throughout the experiments, the gradient surfaces were necessary material to study how the cells are affected by morphogen protein at different concentrations.

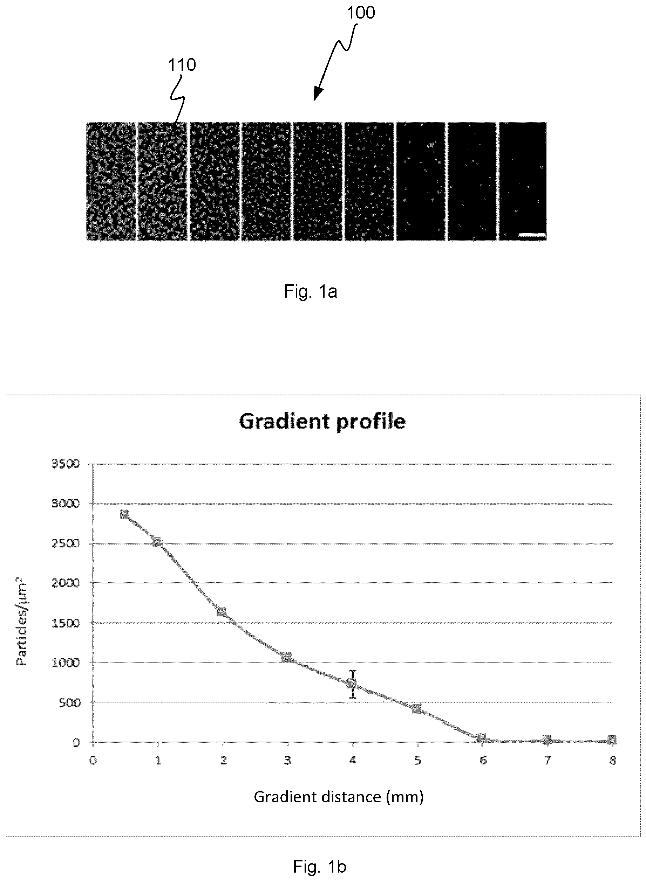

A montage of SEM pictures taken at different positions with 1 mm intervals along the substrate 100 having a gradient surface 110 is provided in Fig 1. A schematic view of such substrate 100 with the gradient surface 110 is provided in Fig. 2a.

Fig. 2e shows a schematic illustration of how the morphogens 80 are attached to the gold nanoparticles 20 in a gradient pattern via the linker streptavidin 60 and with laminin 40 between particles to ameliorate cell adhesion.

As described above, after functionalization of the gradients, seeding and differentiation of c-iPSCs, the gradient surfaces were scanned to visualize the cells. New h.d. surfaces having particle densities in the range where c-iPSCs had shown favourable response were produced, based on the cell response found on the gradient surfaces.

The resulting cell behaviour from gradient surfaces and h.d. surfaces functionalized with morphogen proteins and laminin are presented below.

### Differentiation on gradient surfaces and h.d. surfaces, and c-iPSC analysis on gradient surfaces and h.d. surfaces

### Method - differentiation on surfaces

Each gradient surface and h.d. surface was seeded with 50 000 cells/cm² and incubated in 6 well plates at 37 °C in a humidity chamber for two hours. Thereafter, 2 ml maintenance medium was applied to each well. The maintenance medium was a commercially available medium (Cellartis® DEF-CS™ 500 Basal Medium with Additives, Cellartis, Sweden).

The next day, 2 ml chondrocyte differentiation medium, a previously published modified medium (Nguyen, Hagg et al. 2017) (Table 1), was added with different compositions depending on which morphogen the gradient surface was functionalized with.

To gradient surfaces functionalized with TGFβ-1, TGFβ-3 was added in the differentiation medium.

To gradient surfaces functionalized with TGFβ-3, TGFβ-1 was added to the differentiation medium.

GDF5 gradient surfaces had both TGFβ-1 and TGFβ-3 added in the differentiation medium.

The differentiation medium was changed every second day during the five-day differentiation period.

### Method - c-iPS cell analysis

In order to analyse a middle step of the differentiation process, the results from the differentiation on the surfaces (both controls, gradient surfaces and h.d. surfaces) were analysed. Hence, this method step is not necessary to differentiate the c-iPSCs to chondrocytes, but serves to provide insight into the differentiation process in the first place.

Previous preliminary studies performed by the inventors using c-iPSCs and chondrocytes for differentiation on gradient surfaces showed that especially chondrocytes were difficult to maintain attached to the gradient surface after five days. Therefore, after five days of differentiation on the gradient surfaces in differentiation medium, the cells were fixed with HistofixTM (Histolab Products AB, Sweden), nuclei were stained with 4',6-diamidino-2-phenylindole (DAPI) (ProLongTM Gold Antifade Mountant with DAPI, Thermo Fisher Scientific, USA), and the surfaces were mounted on glass slides. Cell analysis was performed using the fluorescence imaging microscope IN Cell Analyzer 6000 (IN Cell Analyzer 6000, GE Healthcare, United Kingdom), Eclipse 90i (Eclipse 90i, Nikon Instruments, Japan) and Confocal microscope (Nikon A1, Nikon Instruments, Japan).

The results from the cell analysis for the different surfaces produced are presented below.

**Table 1 - Differentiation medium¹**

| Component | **Concentration** | **Company** |
|---|---|---|
| DMEM, high glucose, L-Glutamine and HEPES. PEST added | 4500 mg/L | Gibco |
| Insulin-Transferrin-Selenium | 1/0.55/0.00067 g/L | Thermo Fisher Scientific |
| Ascorbic acid | 14 µg/ml | Sigma Aldrich |
| Dexamethasone | 0.1 µM | Merck |
| Linoleic acid | 5 µg/ml | Sigma Aldrich |
| Sodium Pyruvate | 1 mM | Sigma Aldrich |
| TGFβ-1 | 10 ng/ml | R&D Systems |
| TGFβ-3 | 10 ng/ml | R&D Systems |

| | | |
|---|---|---|
| *Components and concentrations of the used for differentiation of c-iPS cells and for pellet culture. The first six components were always included in the medium. TGFβ-1 and TGFβ-3 were added to the medium dependent on which molecule was functionalized on the surface. TGFβ-1 was not included in the medium for a TGFβ-1 gradient*/*surface and TGFβ-3 was not included in the medium for a TGFβ-3 gradient*/*surface. Both TGFβ-1 and TGFβ-3 were added in the medium for GDF5 gradients*/*surfaces.* | | |

### Results

### Laminin control gradient surfaces

Laminin gradient surfaces (without morphogens) were used as controls to compare against the cellular responses from the morphogen gradient surfaces and h.d. surfaces. Cells on all replicated laminin gradient surfaces resulted in formation of condensed semi-circular structures consisting of cell free cavities, as shown in areas A1 and A2 in Fig. 3).

### GDF5 gradients and h.d. surfaces

Compared to results on laminin control gradient surfaces, c-iPSCs differentiated on GFD5 gradient surfaces resulted in another type of cell behaviour, condensed cell clusters/aggregates (termed *budding zones*), at a particle density of 500 to 1500 particles/µm² (Fig. 4), determined using SEM (Figs 1a and 1b). Larger cell masses were observed over the entire surface, but in the budding zone, small cell budding clusters were formed.

The differentiation process of iPSCs into chondrocytes is initiated by mesenchymal condensation, meaning reduction of intercellular spaces and is followed by continued differentiation. Cellular condensation is an important part of the differentiation process during chondrogenesis and skeletogenesis. To achieve a condensation process, the composition of the cellular microenvironment is crucial (Tacchetti, Tavella et al. 1992).

New homogenous density surfaces (h.d. surface) with a particle density in the range of the budding zone (500 to 1500 particles/µm²) was used to create surfaces with a homogenous GDF5 density (Fig. 5). Fig. 5A shows a GDF5 h.d. surface with a particle density at 400 particles/µm², i.e. lower than the particle density at the observed budding zone. As seen in Fig. 5B, the same cell budding phenomena as observed on the gradient surfaces was also observed on an h.d. surface having a particle density of 900 particles/µm². For comparison, on the h.d. surfaces with the lower particle density (400 particles/µm²) (Fig. 5A), cells formed larger gatherings as well but no indications of budding formations.

### Conclusion

The result found for the GDF5 gradient surfaces was especially noteworthy, where cell budding zones were observed in a specific density range (500 to 1500 particles/µm³), implicating the role of GDF5 at a certain concentration in a condensing budding formation. As described above, such condensation and budding formation is an indicative factor of that chondrogenesis has been induced.

The results were affirmed and repeated on specific h.d. surfaces where the cells behaved in the same way as observed on gradient surfaces, generating budding areas and a condensed structure. The appearance of cell clusters on both GDF5 gradient surfaces and h.d. surfaces provide important information for inducing the condensation and budding formation in chondrocyte differentiation, indicating an early stage in the formation of condensed chondrogenic structures in c-iPSCs. These results suggest that the cell budding responses are dependent on specific GDF5 concentrations and that the particular density interval was essential to induce cellular clusters.

### TGFβ-1 gradients and h.d. surfaces

c-iPSCs on all replicated TGFβ-1 gradient surfaces formed semi-circular structures in areas where the particle density was low, a behaviour comparable to control gradient surfaces with only laminin. At higher TGFβ-1 densities, this cell response was not observed. The cell responses were studied further on h.d. surfaces without a gradient, where separate surfaces with low and high particle density were used to compare the cellular responses when exposed to different single morphogen densities. At low TGFβ-1 h.d. surfaces (600 particles/µm²), cells tended to form the mentioned semi-circular structures consisting of cell free cavities covering the h.d. surfaces (Fig. 5C). At high TGFβ-1 h.d. surfaces (2000 particles/µm²), the cells were evenly distributed over the h.d. surfaces (Fig. 5D).

### Conclusion

It has been suggested that TGFβ, as well as GDF5, induce condensation (Wang, Rigueur et al. 2014). Surprisingly, c-iPSCs grown on TGFβ-1 gradient surfaces did not show condensational tendencies and did not respond similar to cells stimulated with GDF5 as no budding zone was observed on the TGFβ-1 gradient surfaces. Since TGFβ-1 is suggested as relevant to condensation specifically, the lack of budding was somewhat surprising.

At lower TGFβ-1 densities along the gradient surface, cells tended to form another structure in the shape of smaller clusters in a semicircular shape. However, this is more likely due to the effect of laminin. The low h.d. surfaces (600 particles/µm²) resulted, as expected from results from gradient surfaces, in that cells formed cell-free cavities, while cells on high h.d. surfaces (2000 particles/µm²) were evenly distributed, and no cavities were formed.

As cells on all replicated laminin gradient surfaces induced the same pattern seen at lower TGFβ-1 densities, it is most likely that low TGFβ-1 densities are not enough to override the effect of laminin. At higher densities, the effect of TGFβ-1 takes over and the cell uniformity is evident.

### TGFβ-3 gradients and h.d. surfaces

c-iPSCs on TGFβ-3 gradient surfaces did not generate any visual cell response. To compare and confirm the results, h.d. surfaces were used at high and low morphogen densities. Cells were evenly distributed over the gradient surfaces as well as the h.d. surfaces throughout the differentiation both at low (600 particles/µm²) and high (1900 particles/µm²) density (Fig. 5E and5F). No budding zone was observed and no tendency to otherwise condense.

### Conclusion

Results from gradient surfaces with TGFβ-3 did not show any indications of budding formation or condensed structures. Thus, the conclusion is that TGFβ-3 is not as relevant as GDF5 for condensation and to generate budding zones.

As shown in Figs. 5E and 5F, the h.d. surfaces with a high (1900 particles/µm²) and a low (600 particles/µm²) particle density showed no specific cell responses such as observed primarily for GDF5, but also for TGFβ-1. In comparison to TGFβ-1, low densities of TGFβ-3 were not overridden by laminin and no cavities were formed.

Overall, it may be concluded that no budding formation was observed for either TGFβ-1 or TGFβ-3, and when comparing the three different proteins, (GDF5, TGFβ-1, and TGFβ-3) it was clear that GDF5 induced condensation of cells to a higher extent than TGFβ-1 and TGFβ-3.

GDF5 and TGFβ-1 are both molecules that are involved in cellular condensation (Francis-West, Abdelfattah et al. 1999, Coleman, Vaughan et al. 2013; Kim et al. 2014). However, only on the GDF5 surfaces cells formed condensed budding structures. TGFβ-3 have shown to inhibit cellular condensation, which may correlate to the results in our in vitro setting (Jin et al. 2010). Thus, the similarities between TGFβ-1 and TGFβ-3 were somewhat surprising as well as the lack of similarities between the results for TGFβ-1 and GDF5.

### Immunostaining on c-iPSC seeded gradient surfaces and histological pellet sections Method

c-iPSCs on GDF5 gradient surfaces were stained for the proteins SOX9 and TBX3. Histological pellet sections where stained for TBX3. Also, TGFβ-3 h.d. surfaces were used as controls. A differentiation medium comprising GDF5 was added to the TGFβ-3 h.d. control surfaces. Paraffin embedded histological sections were deparaffinised and dehydrated prior immunostaining. Cells on surfaces and in pellets sections were permeabilised prior to immunostaining using 0.1% tritonX-100 (TritonX-100, Sigma Aldrich, USA) in PBS and incubated in room temperature for 10 minutes.

After permeabilisation, cells were incubated in blocking medium (2% BSA (Bovine Serum Albumin, Sigma Aldrich, USA), 0.1% tritonX-100, 100 mM glycine (Glycin, Riedel-de Haen AG, Germany) dissolved in PBS) for 15 minutes, followed by incubation in 500 µl of diluted specific primary antibody (Table 2) at 4°C overnight.

The next day, cells were rinsed and washed with PBS three times for 3 minutes. Thereafter, cells were incubated with blocking medium for 15 minutes followed by incubation with a secondary antibody for 2 hours in a humidity chamber (Table 2). After three rinsing/washing steps in PBS, each for 3 minutes, the cells and pellet sections were mounted with ProLong™ Gold Antifade Mountant media (ProLong™ Gold Antifade Mountant with DAPI, Thermo Fisher Scientific, USA) and were imaged using fluorescence microscopy (Eclipse 90i, Nikon Instruments, Japan) and confocal microscopy (Nikon A1, Nikon Instruments, Japan).

### Results

The c-iPSC behaviour resulting in a budding zone, solely found on GDF5 coated surfaces, were further studied using immunostaining for specific protein expressions, as described in the method section above.

Transcription factor SOX9 (SOX9) is well known to be an important regulating protein in chondrocyte differentiation, mesenchymal condensations and for formation and secretion of cartilage structural proteins. SOX9 is expressed in chondroprogenitors and differentiated chondrocytes but not in hypertrophic chondrocytes, indicating its importance in early cartilage development (Hino, Saito et al. 2014, Lefebvre and Dvir-Ginzberg 2017). Mutations in the SOX9 gene that leads to altered levels of SOX9 proteins is suggested to be linked to skeletal- and degeneration diseases (Lefebvre and Dvir-Ginzberg 2017). SOX9 is a chondrogenic marker and is therefore expected to be present during differentiation (Hino, Saito et al. 2014, Lefebvre and Dvir-Ginzberg 2017).

T-box transcription factor 3 (TBX3) is considered important for, and significantly expressed during, limb formation in early development (Sheeba and Logan 2017). Mutations, which have resulted in decreased protein expressions, are found to cause developmental defects such as ulnar-mammary syndrome (Bamshad, Le et al. 1999).

Hence, the presence of SOX9 and TBX3 are of relevance when determining if differentiation of stem cells into chondrocytes have taken place.

Immunostaining using selected antibodies (Table 2) visualized expression of SOX9 and TBX3 respectively, in c-iPSCs differentiated on GDF5 gradient surfaces. All cell aggregates contained high SOX9 expressions (Fig. 6).

TBX3 expression was indeed observed to be specifically expressed in the buds found in the budding zone (500 to 1500 particles/µm³), primarily in the nuclei but also in forms of highly stained vesicles around nuclei (Fig. 7).

To validate these elevated levels of TBX3 observed in buds on GDF5 gradient surfaces, h.d. surfaces with a particle density corresponding to the budding zone were used.

For comparison, h.d. surfaces with GDF5 at low particle density, TGFβ-3 at budding zone density, TGFβ-3 surfaces at budding zone density with GDF5 added to the medium, and finally, glass slides coated with Coat-1 without added biomolecules were used as controls.

TBX3 expression on TGFβ-3 surfaces with the particle density from the budding zone were more upregulated compared to all other controls, but still less than results from GDF5 mediated budding zones. This finding seemingly confirms that TBX3 is more expressed at the GDF5 levels of the budding zone, as shown in Fig. 8. Cell condensation is seen to some extent (Fig. 8), but not in the way that budding zones are observed on the GDF5 gradient or h.d. surfaces within the budding zone range (500 to 1500 particles/µm³).

c-iPS histological pellets were also stained for TBX3 using immuno-histochemistry (Fig. 9 A3-6, B3, C3). TBX3 were specifically induced in the area of the pellets with upregulated amounts of GAG.

**Table 2 - Antibodies and isotype control antibodies²**

| **Target** | **Primary antibody** | **Isotype control** | **Secondary antibody** |
|---|---|---|---|
| **TBX3** | Anti-TBX3 Antibody, LS-C133955, Nordic Biosite | Mouse IgG1 kappa Isotype Control, 14-4714-82, eBioscience, USA | Alexa Fluor 546, Invitrogen A11030, ThermoFisher Scientific, USA |
| **TBX3** | TBX3 polyclonal antibody, Invitrogen 42-4800, ThermoFisher Scientific, USA | | Alexa Fluor 594, Invitrogen A-21207, ThermoFisher Scientific, USA |
| **SOX9** | Anti-SOX9, ab76997, Abcam, United Kingdom | normal mouse IgG, sc-2025, Santa Cruz, USA | Alexa Fluor 546, Invitrogen A21133, ThermoFisher Scientific, USA |

| | | | |
|---|---|---|---|
| *²Antibodies and isotype control antibodies used for immunostaining of cells on GDF5 gradients.* | | | |

### Pellet cultures from GDF5 gradient surfaces, GDF5 h.d. surface and chondrocyte control experiments

### Method - GDF5 surface pellets and Chondrocyte pellet control experiments

The method described below was conducted using:
- GDF5 gradient surfaces seeded with c-iPSCs;
- GDF5 h.d. surfaces seeded with c-iPSCs - as control, having nanoparticles in a particle density of 440 particles/µm²;
- GDF5 h.d. surfaces seeded with c-iPSCs - having nanoparticles in a particle density within budding zone range. Such surface had particle densities as disclosed in the section *"Preparation of gradient surfaces of GDF5, TGFβ-1 and TGFβ-3*".

After five days of differentiation on GDF5 gradient surfaces or h.d. surfaces, c-iPSCs were removed from each respective surface using trypsin-EDTA (0.05%) (ThermoFisher Scientific, USA).

The trypsin-EDTA reaction was quenched with human serum and the cells were transferred into 15ml conical tubes and centrifuged for 5 minutes at 1200g in 2ml differentiation medium with addition of both TGFβ-1 and TGFβ-3 (Table 1), to form a three-dimensional (3D) structure (also referred to as a *"pellet"* herein), which were to be further differentiated in a differentiation medium.

Said pellets were then stored in 96 well plates in 200µl differentiation medium (Table 1) in a 37°C incubator with 5% CO₂ and were differentiated for five weeks. The medium was changed every third day.

To compare results from the c-iPSC pellets derived from GDF5 gradient surfaces and GDF5 h.d. surfaces (using the method presented above), two control setups were employed. The aim of the control setups was to compare the results from the three c-iPSC pellets with pellets derived from healthy actual chondrocytes to verify that chondrocytes derived from c-iPSC pellets behaved in the same way as ordinary chondrocytes.

Chondrocytes cultured as described in the paragraph "Cell culturing of chondrocytes for control experiments" were used in the two control setups. In the first control experiment, chondrocytes were seeded to a GDF5 gradient surface and later formed into a pellet with the method described above. In the second control, chondrocytes were formed through a conventional pellet culture method not using a surface or gradient surface but with 5% human serum added to the differentiation medium. Pellets from the two controls were formed using the method described above.

Chondrocyte pellets were differentiated for two weeks since it has been shown that after two weeks, chondrocytes from OA donors have started to express high levels of both collagen type II and proteoglycans (Tallheden, Bengtsson et al. 2005). After the differentiation period, all pellets were fixated with HistofixTM (Histolab Products AB, Sweden) overnight and then stored in 70% ethanol.

Thereafter, all pellets were sent to HistoCenter (Mölndal, Sweden) for sectioning and staining with Alcian Blue van Gieson to visualize the extracellular matrix (ECM) composition of the pellets. The pellet compositions were analysed using a light microscope (Eclipse 90i, Nikon Instruments, Japan). Histological sections were stained with Alcian Blue van Gieson and displayed areas with a high amount of both blue colored glycosaminoglycans (GAGs) and red/purple coloured collagen.

### Results

As described in the method section above, cells were pre-differentiated on GDF5 gradient surfaces and GDF5 h.d. surfaces (one particle density from the range of the budding zone, 500 to 1500 particles/µm², and one density with a lower particle density as a control, 400 particles/µm²) before being cultured as 3D micromass pellets suspended in differentiation medium (Table 1). A control having a particle density higher than 1500 particles/µm² was not tested partly due to that at higher concentrations that 1500, cells have tendency to come off the substrate. As can be seen in Fig. 4, cell growth is present also at higher particle densities, however, no budding formation if observed. Hence, on an h.d. surface, cells have difficulty to attach to the surface as a result of the higher particle densities causing such reference/control specimen to be unfit for control experiments.

The blue colour was mainly concentrated to one area of the pellets while the red/purple was expressed around the blue area and mostly in the distinct edges.

Two different chondrocyte pellets were used as controls. One using a GDF5 gradient surface and the same differentiation medium as the c-iPS pellets (Table 1) (Fig. 9 D1 & D2) and the other without GDF5 gradient or h.d. surface but the addition of 5% human serum to the medium (Fig. 9 E1 & E2). Both chondrocyte control pellets seem to, compared to the c-iPS pellets, have a more compact pellet structure. However, according to the colouring, the structural components in all pellets are similar.

The coloring of the c-iPS pellets obtained from GDF5 gradient surfaces and h.d. surfaces were compared to chondrocyte control pellets and all indicated the presence of both GAGs (blue) and collagen (red/purple). This implies that the differentiation of the c-iPSC pellets using the protocol including GDF5 gradients and surfaces drives the cells meritoriously towards the chondrocyte linage.

### References

Aumailley, M. (2013). "The laminin family." Cell Adh Migr 7(1): 48-55.
Bamshad, M., T. Le, W. S. Watkins, M. E. Dixon, B. E. Kramer, A. D. Roeder, J. C. Carey, S. Root, A. Schinzel, L. Van Maldergem, R. J. Gardner, R. C. Lin, C. E. Seidman, J. G. Seidman, R. Wallerstein, E. Moran, R. Sutphen, C. E. Campbell and L. B. Jorde (1999). "The spectrum of mutations in TBX3: Genotype/Phenotype relationship in ulnar-mammary syndrome." Am J Hum Genet 64(6): 1550-1562.
Borestrom, C., S. Simonsson, L. Enochson, N. Bigdeli, C. Brantsing, C. Ellerstrom, J. Hyllner and A. Lindahl (2014). "Footprint-free human induced pluripotent stem cells from articular cartilage with redifferentiation capacity: a first step toward a clinical-grade cell source." Stem Cells Transl Med 3(4): 433-447.
Coleman, C. M., E. E. Vaughan, D. C. Browe, E. Mooney, L. Howard and F. Barry (2013). "Growth differentiation factor-5 enhances in vitro mesenchymal stromal cell chondrogenesis and hypertrophy." Stem Cells Dev 22(13): 1968-1976.
Francis-West, P. H., A. Abdelfattah, P. Chen, C. Allen, J. Parish, R. Ladher, S. Allen, S. MacPherson, F. P. Luyten and C. W. Archer (1999). "Mechanisms of GDF-5 action during skeletal development." Development 126(6): 1305-1315.
Goldring M B. Chondrogenesis, chondrocyte differentiation and articular cartilage metabolism in health and osteoarthrosis. Therapeutic Advances in Musculoskeletal Disease. 2012 Aug; 4(4): 269-285.
Hino, K., A. Saito, M. Kido, S. Kanemoto, R. Asada, T. Takai, M. Cui, X. Cui and K. Imaizumi (2014). "Master regulator for chondrogenesis, Sox9, regulates transcriptional activation of the endoplasmic reticulum stress transducer BBF2H7/CREB3L2 in chondrocytes." J Biol Chem 289(20): 13810-13820.
Kim Y I. Ryu J., Yeo J E., Choi Y J., Kim Y S., Ko K. and Koh Y. Overexpression of TGF-β1 enhances chondrogenic differentiation and proliferation of human synovium-derived stem cells. Biochemical and Biophysical Research Communications. Volume 450, Issue 4, 8 August 2014, Pages 1593-1599.
Lefebvre, V. and M. Dvir-Ginzberg (2017). "SOX9 and the many facets of its regulation in the chondrocyte lineage." Connect Tissue Res 58(1): 2-14.
Nguyen, D., D. A. Hagg, A. Forsman, J. Ekholm, P. Nimkingratana, C. Brantsing, T. Kalogeropoulos, S. Zaunz, S. Concaro, M. Brittberg, A. Lindahl, P. Gatenholm, A. Enejder and S. Simonsson (2017). "Cartilage Tissue Engineering by the 3D Bioprinting of iPS Cells in a Nanocellulose/Alginate Bioink."Sci Rep 7(1): 658.
Jin E J., Park K S., Kim D., Lee Y S., Sonn J., Chang J., Bang O., and Kang, S S., TGF-beta 3 inhibits chondrogenesis by suppressing precartilage condensation through stimulation ofN-cadherin shedding and reduction of cRREB-1 expression. Molecules and cells. 29. 2010. 425-32.
Sheeba, C. J. and M. P. Logan (2017). "The Roles of T-Box Genes in Vertebrate Limb Development." Curr Top Dev Biol 122: 355-381.
Tacchetti, C., S. Tavella, B. Dozin, R. Quarto, G. Robino and R. Cancedda (1992). "Cell condensation in chondrogenic differentiation." Exp Cell Res 200(1): 26-33.
Tallheden T., Bengtsson C., Brantsing C., Sjögren-Jansson E., Carlsson L., Peterson L., Brittberg M., and Lindahl A. Proliferation and differentiation potential of chondrocytes from osteoarthritic patients. Arthritis Research & Therapy. 2005; 7(3): R560-R568.
Wang, W., D. Rigueur and K. M. Lyons (2014). "TGFbeta signaling in cartilage development and maintenance." Birth Defects Res C Embryo Today 102(1): 37-51.

## Claims

1. A method for differentiating induced pluripotent stem cells (iPSCs) into chondrocytes, said method comprising the steps of:
- seeding a surface (110) of a substrate (100) with iPSCs, wherein the surface (110) is coated with nanoparticles (20) in a particle density of at least 500 particles/µm², and parts (120) of the surface (110) in between said nanoparticles (20) are coated with a coating agent (40), wherein growth differentiation factor 5 (GDF5) molecules are attached to said nanoparticles (20);
- adding a first differentiation medium to the seeded iPSCs; and
- allowing the seeded iPSCs to differentiate at least into chondrocyte progenitor cells on the surface (110) in the presence of the first differentiation medium.

2. The method according to claim 1, wherein the particle density is less than 1500 particles/µm².

3. The method according to claim 1 or 2, wherein said iPSCs are differentiated for between 3 and 10 days, preferably between 4 and 8 days, on said substrate surface (110).

4. The method according to any one of claims 1 to 3, wherein the method further comprises:
- removing formed condensed chondrocyte progenitor cell aggregates from the substrate surface (110) with GDF5 attached thereto;
- further differentiating the removed condensed chondrocyte progenitor cell aggregates into chondrocytes, in a second differentiation medium.

5. The method according to claim 4, wherein said removed condensed cell aggregates are formed into a three dimensional pellet structure, before being further differentiated.

6. The method according to claim 4 to 5, wherein the step of further differentiating the removed condensed chondrocyte progenitor cell aggregates is performed for 4 to 10 weeks, preferably for 5 to 8 weeks.

7. The method according to any one of the preceding claims, wherein said first and second differentiation medium both comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof.

8. The method according to any one of the preceding claims, wherein a maintenance medium is added to the substrate surface (110) after the iPSCs have been seeded to the surface (110) and before the differentiation medium is added.

9. The method according to any one of the preceding claims, wherein said iPSCs are chondrocyte derived PSCs; preferably the chondrocyte derived PSCs being generated by reprogramming chondrocytes using a method based on mRNA delivery; preferably the method being footprint-free.

10. The method according to any one of the preceding claims, wherein
said coating agent (40) is a proliferative agent, preferably the proliferative agent being an extra cellular matrix (ECM) protein, such as a laminin or fibronectin, a cell specific cytokine or a combination thereof; and/or
wherein the nanoparticles (20) are gold particles coated with thiolated streptavidin, and wherein the GDF5 molecules are biotinylated and attached to the nanoparticles through biotin/streptavidin interaction.

11. The method according to any one of the preceding claims, wherein 20 000 to 100 000 iPS cells/cm², preferably 30 000 to 70 000 iPS cells/cm², more preferred about 50 000 cells/cm², are seeded on the substrate surface (110).

12. The method according to any one of the preceding claims, further comprising a step of integrating the obtained chondrocytes into a matrix/scaffold.

13. A chondrocyte obtainable by the method according to any one of the claims 1 to 11.

14. A solid substrate for differentiating induced pluripotent stem cells (iPSCs) into chondrocytes, comprising:
- a substrate surface (110) coated with nanoparticles (20) linked to said surface (110) in a particle density of at least 500 particles/µm²;
- a coating agent (40) coating the parts (120) of surface (110) in between the nanoparticles on the substrate surface (110); and
- growth differentiation factor 5 (GDF5) molecules attached to said nanoparticles (20).

15. The substrate according to claim 14, wherein
- the particle density is less than 1500 particles/µm²; and/or
- said substrate (100) is made of glass; and/or
- the nanoparticles (20) are gold nanoparticles coated with thiolated streptavidin, and the GDF5 molecules are biotinylated and attached to the gold nanoparticles through biotin/streptavidin interaction; and/or
- the coating agent (40) is a proliferative agent, preferably the proliferative agent being an extra cellular matrix (ECM) protein, preferably a laminin or fibronectin, a cell specific cytokine, or a combination thereof.
